# EUROPÄISCHE PATENTSCHRIFT

(11) **EP 3 401 300 B1**
(45) Veröffentlichungstag und Bekanntmachung des Hinweises auf die Patenterteilung: **23.06.2021**
(21) Anmeldenummer: 17400025.7
(22) Anmeldetag: 12.05.2017
(51) Int. Cl.: C07C 29/152, C07C 31/04, B01J 8/04

(54) **VERFAHREN ZUR HERSTELLUNG VON METHANOL**
METHOD FOR THE PRODUCTION OF METHANOL
PROCÉDÉ POUR LA PRODUCTION DU MÉTHANOL

(43) Veröffentlichungstag der Anmeldung: 14.11.2018
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Ochs, Andreas, 61350 Bad Homburg (DE); Castillo-Welter, Frank, 61381 Friedrichsdorf (DE); Haag, Stéphane, 60598 Frankfurt am Main (FR); Frind, Robert, 64331 Weiterstadt (DE); Schuhmann, Timm, 63067 Offenbach (DE); Wagner, Marc, 94100 Saint Maur des Fosses (FR); Valentin, Solene, 92190 Meudon (FR)
(74) Vertreter: Dropsch, Holger

(56) Entgegenhaltungen:
- EP-A1- 0 682 002
- EP-A1- 0 790 226
- EP-A1- 3 219 697
- EP-A2- 0 483 919
- EP-A2- 0 494 350
- WO-A1-2005/115607
- WO-A1-2012/139703
- WO-A2-01/17936
- DE-A1-102008 049 622

## Beschreibung

### Gebiet der Erfindung

Die Erfindung betrifft ein Verfahren zum Durchführen exothermer Gleichgewichtsreaktionen, insbesondere für die Durchführung der Methanolsynthese durch heterogen-katalysierte Umsetzung von Wasserstoff und Kohlenoxide umfassendem Synthesegas an festen Katalysatoren.

### Stand der Technik

Verfahren zur Durchführung exothermer Gleichgewichtsreaktionen sind der Fachwelt seit langem bekannt. Eine industriell besonders wichtige Reaktion dieses Typs ist die Methanolsynthese durch heterogen-katalytische Umsetzung von Synthesegas, also Gemischen von Wasserstoff und Kohlenoxiden. In Ullmann's Encyclopedia of Industrial Chemistry, Sixth Edition, 1998 Electronic Release, Kapitel "Methanol", Unterkapitel 5.2 "Synthesis" werden verschiedene Grundverfahren zur Herstellung von Methanol durch katalytische Umsetzung von Wasserstoff und Kohlenstoffoxide enthaltendem Synthesegas beschrieben, bei denen solche Reaktoren eingesetzt werden.

Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern. Diese Maßnahme wird auch in der deutschen Offenlegungsschrift DE 2934332 A1 und der europäischen Patentanmeldung EP 1016643 A1 gelehrt.

In der wassergekühlten Reaktorstufe wird üblicherweise der Hauptumsatz des Synthesegases (CO, CO2, H2) erzielt und der größte Teil der Reaktionswärme abgeführt, während in der gasgekühlten Stufe bei milderen Bedingungen ein dennoch erheblicher Teil des Synthesegases umgesetzt wird.

In manchen Anlagenkonfigurationen wird zusätzlich eine Zwischenkondensationsstufe zwischen den beiden Reaktionsstufen vorgesehen, um den Anteil der gebildeten Reaktionsprodukte (vorwiegend Methanol und Wasser) im Einsatzgas zur zweiten Reaktionsstufe zu verringern und damit den erzielbaren Umsatz der Edukte weiter zu erhöhen. Eine solche Anlagenkonfigurationen wird beispielsweise in der deutschen Patentschrift DE 10 2008 049 622 B4 gelehrt.

Bei dem wassergekühlten Reaktor (WCR) handelt es sich üblicherweise um einen Röhrenreaktor mit entsprechenden Rohrplatten, bei dem der Katalysator in die Rohre gefüllt wird, während die Kühlung mittels siedendem Wasser bzw. Dampferzeugung auf der Mantelseite um die Rohre erfolgt. Im gasgekühlten Reaktor (GCR) erfolgt die Kühlung mit dem Einsatzgas, welches durch die Rohre geführt wird und sich auf dem Weg zur ersten Reaktionsstufe (WCR) erwärmt, während der Katalysator um die Rohre gefüllt wird und die Reaktion auf der Mantelseite des GCR stattfindet. Die Reaktionsstufen sind hinsichtlich ihrer Nennweite mit großen bzw. sehr großen Rohrleitungen verbunden; je nach Anlagenkapazität sind Rohrdurchmesser von bis zu 1 m möglich. Dies ist vor allem auf die großen Gasmengen zurückzuführen, die zu der zweiten Stufe zurückgeführt (Recycle-Gas) und dem Frischgas, also frischem Synthesegas aus der Gaserzeugung, zugemischt werden. Das sich ergebende Gasgemisch aus Rückführungsgas und Frischgas wird nach erfolgter Vorwärmung im GCR der ersten Reaktionsstufe (WCR) zugeführt. Die Rückführungsgasmenge (Recycle-Gas) ist üblicherweise deutlich größer als die Frischgasmenge und ist vom erzielten Umsatz in der Reaktorsektion abhängig. Das Rückführverhältnis RR (RR=R/F) aus Rückführungsgasmenge (R) zu Frischgasmenge (F) liegt oft über 2 und in einigen Fällen sogar über 3,5. Je niedriger der Umsatz von Synthesegas durch die Reaktorsektion per Durchgang ist, um so höher ist das erforderliche Rückführverhältnis RR, um eine hinreichende Ausbeute zu erzielen.

Damit erhöht sich die umlaufende Gasmenge entsprechend, was die Belastung der Reaktoren erhöht und größere Rohrnennweiten der verbindenden Rohrleitungen bedarf und auch zu einem höherem Bedarf an Kompressionsenergie (höhere Durchflussmenge und Druckverlust) führt.

Das Patentdokument WO 2012/139703 A1 offenbart ein Verfahren zur Herstellung von Methanol aus inertenreichem Synthesegas, bei dem dem ein- oder mehrstufigen Synthesekreislauf ein katalytischer Vorreaktor vorgeschaltet wird, in dem ein erster Teil des Synthesegases zu Methanol umgewandelt wird. Ferner wird dem Synthesekreislauf eine Inertgasabtrennungsstufe, beispielsweise eine Druckwechseladsorptionsanlage ode eine Membrananlage, nachgeschaltet, wodurch ein an Wasserstoff angereicherter Synthesegasstrom in den Synthesekreislauf zurückgeführt werden kann. Bei der Verarbeitung methanreicher Synthesegase kann die Inertgasabtrennungsstufe auch einen Autothermreformer umfassen, in dem Methan zu Kohlenoxiden und Wasserstoff umgesetzt wird, die ebenfalls in den Synthesekreislauf zurückgeführt werden.

Bei dem in Patentdokument EP 0 682 002 A1 offenbarten Verfahren wird Methanol aus einem Wasserstoff und Kohlenoxide enthaltenden Synthesegas an kupferhaltigen Katalysatoren bei Drücken im Bereich von 20 bis 120 bar und Temperaturen im Bereich von 200 bis 350°C erzeugt. Man leitet das Synthesegas zunächst durch einen ersten Synthesereaktor, der als Schachtreaktor ausgebildet ist und eine Schüttung eines kupferhaltigen Katalysators enthält. Die Umsetzung im Schachtreaktor erfolgt adiabatisch und ohne Synthesegas-Kreislauf. Das im ersten Synthesereaktor nicht umgesetzte Gasgemisch wird zusammen mit Kreislaufgas durch einen zweiten Synthesereaktor geflihrt, der einen in Röhren angeordneten, durch siedendes Wasser indirekt gekühlten kupferhaltigen Katalysator aufweist. Vorzugsweise werden 10 bis 30 % der Kohlenoxide des Synthesegases im Schachtreaktor zu Methanol umgesetzt.

Das Patentdokument EP 0 494 350 A2 offenbart ein Verfahren und einer Anlage zur Methanolsynthese aus Wasserstoff, Kohlenmonoxid und Kohlendioxid unter Druck mit wenigstens einer Stufe, die einen Reaktor enthält. Mit diesem Verfahren soll die Ausbeute erhöht und die Regelungsmöglichkeit verbessert werden. Dies wird dadurch erreicht, dass wenigstens zwei Synthesestufen mit je einem Reaktor eingesetzt werden und die letzte Synthesestufe mit einem Kreislaufgas-Verdichter ausgerüstet ist, wobei die vorgeschalteten Synthesestufen zur Bildung von Teilkreislaufen über diesen Kreislaufgas-Verdichter beaufschlagt werden.

Das Patentdokument WO 01/17936 A2 offenbart ein Verfahren zur Methanolsynthese aus Wasserstoff, Kohlenmonoxid und Kohlendioxid unter Druck mit wenigstens einer Synthesestufe, die wenigstens einen katalysatorgefüllten Methanolreaktor enthält, wobei bei mehreren Stufen wenigstens eine Stufe mit einem Verdichter ausgestattet ist. Mit diesem Verfahren und einer entsprechenden Anlage sollen bestehende Nachteile des Stands der Technik überwunden und insbesondere die Möglichkeit geschaffen werden, Wärmetauscherfläche einzusparen und/oder die Ausbeute unter besonderer Berücksichtigung der veränderlichen Katalysatoraktivitat zu erhöhen. Dies wird verfahrensmäßig dadurch erreicht, dass in zumindest einer Synthesestufe das für den Einsatz in einem katalysatorgefiillten Methanolreaktor bestimmte Gasgemisch direkt vor Zugabe in besagten Methanolreaktor in einem zusatzlichen Trimmerhitzer aufgeheizt wird, wobei sich eine entsprechende Anlage dadurch auszeichnet, dass in wenigstens einer Synthesestufe ein Trimmerhitzer (5) zur Erwärmung des den Methanolreaktor (10) unmittelbar beaufschlagenden Gasgemisches vorgesehen ist. Gemäß der Offenbarung des Patentdokuments EP 0 483 919 A2 wird Methanol in einem Verfahren hergestellt, das dadurch gekennzeichnet ist, dass ein gasförmiges Gemisch, das Wasserstoff und Kohlenmonoxid enthält, in Gegenwart eines Katalysators in einer Vielzahl von aufeinanderfolgend angeordneten Stufen umgesetzt wird, wobei das gasförmige Gemisch den Katalysator in jeder Stufe in einem Wirbelschichtbereich kontaktiert, während es gekühlt wird, wobei in diesem Verfahren Methanol aus dem Reaktionsgemisch zwischen aufeinanderfolgenden Stufen entfernt wird. Die Entfernung des Methanols zwischen den Stufen erfolgt vorzugsweise durch Kühlen des Ausflussstroms aus dem vorhergehenden Reaktor und ermöglicht die Kondensation des Methanols. Die Abkühlung des Abgasstroms erfolgt vorzugsweise durch Wärmeaustausch mit kaltem Speisegas, vorzugsweise unter Verwendung eines Wärmetauschers mit einer spezifischen Wärmeübertragungsfläche von mindestens 150 m²/m³, vorzugsweise von mindestens 200 m²/m³.

Das Patentdokument WO 2005/115607 A1 betrifft ein Verfahren zur Durchführung heterogen katalytischer exothermer Gasphasenre aktionen bei erhöhter Temperatur und erhöhtem Druck, wobei man das Synthesegas, bestehend aus einem Gemisch von Frischgas und/oder Umlaufgas, durch mindestens zwei in einem Synthesesystem in Serie geschaltete Synthesestufen leitet, wobei die Produktgase aus den Synthesestufen, mit Ausnahme der letzten Stufe, in mindestens zwei Teilströme getrennt werden, wobei ein Teilstrom soweit gekühlt wird, bis Produkt auskondensiert und das produkthaltige Kondensat von den gasförmigen Bestandteilen getrennt wird, und anschließend die gasförmigen Bestandteile mit dem warmen Teil des Produktgases zusammengeführt werden, um die Eintrittstemperatur der nächsten Synthesestufe zu erreichen. Weiterhin kann der Teilstrom, aus dem Produkt auskondensiert und entfernt wurde, vor seiner Wiederzumischung zum warmem Teil des Produktgases erwärmt werden, wobei die Temperatur nach der Erwärmung unter der des warmem Teils des Produktgases liegt und die Wärme, die zur Erwärmung des Teilstroms, der aus dem Produkt auskondensiert wurde, genutzt wird, diesem Teilstrom bei seiner Abkühlung wenigstens teilweise entzogen wird.

Es kann festgestellt werden, dass trotz der Vielzahl der im Stand der Technik vorgeschlagenen technischen Lösungen weiterhin Bedarf an Verfahren zum Durchführen exothermer Gleichgewichtsreaktionen, insbesondere für die Durchführung der Methanolsynthese durch heterogen-katalysierte Umsetzung von Wasserstoff und Kohlenoxide umfassendem Synthesegas an festen Katalysatoren besteht, die eine verbesserte Kontrolle und Steuerung der Reaktion in den einzelnen Synthesestufen gestatten.

### Beschreibung der Erfindung

Die Aufgabe der vorliegenden Erfindung besteht daher darin, ein Verfahren anzugeben, das die beschriebenen Nachteile der aus dem Stand der Technik bekannten Verfahren nicht aufweist und das insbesondere einen hohen Umsatz bezüglich der Zielprodukte der exothermen Reaktion sowie die Möglichkeit bietet, die Reaktionsbedingungen entlang der Längskoordinate des Reaktors nachzuführen und somit zu optimieren, was beispielsweise im Falle der Methanolsynthese zu einer Reduzierung des Rückführverhältnisses auf kleinere Werte führt, wie sie bei Verwendung der aus dem Stand der Technik bekannten Verfahren bekannt sind.

Diese Aufgabe wird durch ein Verfahren mit den Merkmalen des Anspruchs 1 gelöst. Weitere Ausgestaltungen der Erfindung ergeben sich aus den Unteransprüchen.

### Erfindungsgemäßes Verfahren:

Verfahren zum Herstellen von Methanol durch Umsetzen eines Wasserstoff und Kohlenoxide enthaltenden Synthesegaseinsatzes, umfassend folgende Verfahrensschritte:
(a) Bereitstellen eines Reaktors, umfassend mehrere Reaktionszellen in einem Reaktormantel, wobei jede Reaktionszelle die folgenden, hintereinander geschalteten und in Fluidverbindung miteinander stehenden Baugruppen umfasst:
   (aa) eine Vorwärmzone, geeignet zum Aufheizen des Einsatzgemischs oder des gasförmigen Produktstroms aus der vorgelagerten Reaktionszelle, wobei die Vorwärmzone in der ersten Reaktionszelle in Strömungsrichtung des gasförmigen Einsatzgemischs optional entfallen kann,
   (ab) mindestens eine Reaktionszone, enthaltend einen für die durchzuführende exotherme Gleichgewichtsreaktion aktiven Katalysator als Katalysatorschüttung in einem Katalysatorbett und eine mit dem Katalysatorbett in einer Wärmeaustauschbeziehung stehende Kühlvorrichtung,
   (ac) mindestens eine Kühlzone, enthaltend eine Kühlvorrichtung, geeignet zur Abkühlung des aus der Reaktionszone austretenden, teilumgesetzten und mit kondensierbarem Reaktionsprodukt beladenen, gasförmigen Produktstroms auf eine Temperatur unterhalb des Taupunktes dieses Gases,
   (ad) eine Abscheidezone, enthaltend eine Phasentrennvorrichtung zum Auftrennen des aus der Kühlzone austretenden Produktstroms in einen von Kondensat befreiten, gasförmigen Produktstrom und einen flüssiges Reaktionsprodukt umfassenden Kondensatstrom,
   (ae) Mittel zum Ausleiten des flüssiges Reaktionsprodukt umfassenden Kondensatstroms und optional Mittel zum Zuführen des Kondensatstroms zu einer Aufarbeitungsvorrichtung für das Reaktionsprodukt,
   (af) Mittel zum Ausleiten des von Kondensat befreiten, gasförmigen Produktstroms und Mittel zum Zuführen dieses gasförmigen Produktstroms zu einer nachgelagerten, stromabwärts angeordneten Reaktionszelle oder Mittel zum Ausleiten des gasförmigen Produktstroms aus dem Verfahren,
(b) Bereitstellen eines Wasserstoff und Kohlenoxide enthaltenden Synthesegaseinsatzes und Einleiten desselben in den Reaktor,
(c) Mindestens teilweises Umsetzen des Synthesegaseinsatzes in dem Reaktor unter Methanolumsetzungsbedingungen ,
(d) Ausleiten eines Methanol und Wasser umfassenden, flüssigen Reaktorproduktstroms aus dem Reaktor und optional Zuführen des flüssigen Reaktorproduktstroms zu einer weiteren Abscheidevorrichtung und/oder mindestens einer weiteren Methanol-Aufarbeitungsvorrichtung,
(g) Ausleiten eines Synthesegas-Abstroms und Rückführen dieses Synthesegas-Abstroms zu dem Reaktor mit einem festgelegten Rückführverhältnis und/oder Ausleiten des Synthesegas-Abstroms aus dem Verfahren.

Unter Fluidverbindung zwischen zwei Bereichen des erfindungsgemäßen Reaktors wird dabei jegliche Art von Verbindung verstanden, die es ermöglicht, dass ein Fluid, beispielsweise der Einsatzgasstrom oder der Synthesegasproduktstrom, von dem einen zu dem anderen der beiden Bereiche strömen kann, unbeachtlich etwaiger zwischengeschalteter Bereiche oder Bauteile.

Mit Wärmeaustauschbeziehung ist die Möglichkeit des Wärmeaustauschs oder der Wärmeübertragung zwischen zwei Bereichen des erfindungsgemäßen Reaktors gemeint, wobei alle Mechanismen des Wärmeaustauschs oder der Wärmeübertragung wie Wärmeleitung, Wärmestrahlung oder konvektiver Wärmetransport zum Tragen kommen können. Unter einer indirekten Wärmeaustauschbeziehung wird dabei insbesondere die Art des Wärmeaustauschs oder der Wärmeübertragung verstanden, die durch eine Wand hindurch erfolgt (sog. Wärmedurchgang), der die Etappen des Wärmeübergangs von Fluid 1 auf die Oberfläche der Wand, der Wärmeleitung durch die Wand und des Wärmeübergangs von der Oberfläche der Wand auf Fluid 2 umfasst.

Unter Methanolumsetzungsbedingungen sind die dem Fachmann an sich bekannten Verfahrensbedingungen, insbesondere von Temperatur, Druck und Verweilzeit, zu verstehen, wie sie oben beispielhaft genannt und detailliert im einschlägigen Schrifttum erörtert werden und bei denen mindestens ein Teilumsatz, bevorzugt allerdings technisch relevante Umsätze der Edukte CO bzw. CO₂ und Wasserstoff zum Produkt Methanol erfolgt. Entsprechend wird unter einem für die Methanolsynthese aktiven Katalysator ein Katalysator verstanden, der unter Methanolumsetzungsbedingungen eben solche Umsätze bewirkt.

Unter Mitteln zum Einleiten, Ausleiten usw. werden alle die Vorrichtungen, Vorrichtungsbestandteile, Baugruppen und Bauteile verstanden, die es ermöglichen, dass das betreffende Fluid den betrachteten räumlichen Bereich, beispielsweise einen Behälter, verlässt. Hierunter werden insbesondere Rohrleitungen, Pumpen, Verdichter, sonstige Fördervorrichtungen sowie die entsprechenden Durchtrittsöffnungen in der Behälterwand verstanden.

Unter der katalytischen Aktivität, insbesondere im Zusammenhang mit einer unterschiedlichen katalytischen Aktivität beim Vergleich zweier unterschiedlicher Katalysatoren, wird der erzielte Umsatzgrad pro Längeneinheit des Katalysatorbetts von Edukten zu Produkten verstanden. Die Aktivität wird beeinflusst von der chemischen Zusammensetzung, Dotierung, Vergiftung, verfügbaren Oberfläche etc. des Katalysatormaterials, aber auch durch die Geometrie der Katalysatorpartikel und texturellen Parametern des Katalysatorbetts, z. B. dessen Porosität oder Packungsdichte. Aufgrund der Exothermie der betrachteten Reaktionen korreliert eine hohe katalytische Aktivität mit einer hohen Wärmefreisetzung pro Längeneinheit des Katalysatorbetts.

Die in Anspruch 1. (aa) erwähnte Option, dass die Vorwärmzone in der ersten Reaktionszelle in Strömungsrichtung des gasförmigen Einsatzgemischs entfallen kann, wird insbesondere dann realisiert werden, wenn eine außerhalb des erfindungsgemäßen Reaktors angeordnete und diesem vorgeschaltete Heizvorrichtung vorhanden ist, die die Einstellung der Reaktionstemperatur vor Eintritt in die erste Reaktionszone gewährleistet.

Der Erfindung liegt die Erkenntnis zugrunde, dass durch eine optimale Temperaturführung sowie wiederholtes Abführen von Produkten aus der Reaktionszone die Produktionsraten bzw. Raum-Zeit-Ausbeuten entlang des Reaktionsweges deutlich verbessert werden können, Das Temperaturprofil entlang des Reaktionsweges wird durch den Einsatz eines mehrstufigen Reaktionssystems erheblich verbessert, wodurch ein deutlich höherer Umsatz per Durchgang erzielt wird.

Auch die Nebenproduktbildung bei der Methanolsynthese verringert sich bei der Durchführung des erfindungsgemäßen Verfahrens gegenüber dem Stand der Technik.

Ein verbessertes Temperaturprofil im Reaktor lässt sich grundsätzlich auch mit Hilfe eines Katalysator-Layer-Managements erzielen. Dabei würde man in dem Bereich, in dem man den höchsten Umsatz (Exothermie) und damit die höchsten Temperaturen erwartet, einen weniger aktiven Katalysator einsetzen und in Bereichen, wo weniger Umsatz erwartet wird, einen aktiveren Katalysator einsetzen. Jedoch ist ein solches Katalysator-Layer-Management relativ unflexibel, da man die verschiedenen Katalysatorlagen auf Basis einer bestimmten Katalysatoraktivität und einer entsprechenden Gaszusammensetzung auswählen und festlegen muss. Die Katalysatoraktivität verändert sich jedoch über seine Laufzeit der Syntheseanlage durch seine fortschreitende Desaktivierung.

Das Layer-Management und die zugehörige Kühlung des Reaktionsbettes müssen aufeinander abgestimmt werden. Während der Katalysatorlaufzeit und der damit verbundenen Katalysatordesaktivierung ändern sich die Bedingungen und eine Anpassung der Reaktionstemperatur und der zugehörigen Kühlung/Kühltemperatur ist wünschenswert, um die Desaktivierung zumindest teilweise zu kompensieren und einen hohen Umsatz bei geringer Nebenproduktbildung sicherzustellen. Mit den aus dem Stand der Technik bekannten Reaktoren lässt sich eine Anpassung der Kühlung nur für den gesamten Reaktor vornehmen; üblicherweise desaktivieren aber nicht alle Katalysatorschichten über die Betriebszeit in gleichem Maße. Die Einstellung spezifischer Reaktionsbedingungen stellt daher immer einen Kompromiss dar.

Mit dem erfindungsgemäßen Ansatz können die Reaktionsbedingungen in den verschiedenen Reaktionszellen dagegen individuell in Abhängigkeit der Katalysatoraktivität, der Gaszusammensetzung in jeder Stufe auch über die Laufzeit angepasst werden. Damit werden ein hoher Umsatz und eine geringe Nebenproduktbildung in den verschiedenen Reaktionszellen erreicht.

Mit der optimierten Temperaturführung werden auch die Maximaltemperaturen (sowie Temperaturspitzen, sog. Hot-Spots) im Katalysatorbett reduziert. Dies wirkt sich neben der Ausschleusung des Koppelproduktes aus dem Reaktionssystem, beispielsweise von Wasser bei der Methanolsynthese, positiv auf die Katalysatorlaufzeit aus. Es ist bekannt, dass sowohl hohe Temperaturen im Katalysatorbett als auch hohe Wasserkonzentrationen im Reaktionsgas zu einer rascheren Katalysatordesaktivierung beitragen.

Mit dem vorgeschlagenen Konzept wird eine verbesserte Raum-Zeit-Ausbeute erzielt; somit kann auch die Rückführungsgasmenge (Gaskreislauf) erheblich reduziert werden. Grundsätzlich kann der Reaktor somit verkleinert werden und auch der Druckverlust reduziert sich. Durch die Reduzierung der Rückführungsgasmenge ergibt sich auch, dass die Menge und die Konzentration an akkumulierten Inertgasen, beispielsweise nicht umgesetztes Methan aus der Synthesegas-Erzeugung, im Synthesegas-kreislauf deutlich verringert werden und damit den gesamten Methanol-Synthesekreislauf mit Reaktorstufen, Kreislaufverdichter und weiteren Ausrüstungen entlastet. Als Optimum kann man den Vollumsatz pro Reaktordurchgang ansehen, bei dem auf einen Synthesegaskreislauf vollständig verzichtet werden könnte und mithin keine Ackumulierung von Inertgasen mehr auftritt. Ein derartiger Ansatz ist auch für andere Einsatzgaszusammensetzungen mit hohen Inertgasanteilen (z, B, hoher Stickstoffanteil bei Synthesegaserzeugung unter Verwendung von Luft) von besonderem Interesse, da im Synthesegaskreislauf die im Kreis zu führende Inertgasmenge ansteigt.

Durch kontrolliertes Abscheiden der flüssigen Produkte sowie Temperaturkontrolle in den einzelnen Reaktionszellen wird ein Auskondensieren im Katalysatorbett vermieden und der Katalysator geschont.

Die abgeschiedenen Kondensate mit unterschiedlichen Methanolanteilen können bei unterschiedlichen Bedingungen aufgereinigt bzw. direkt als Einsatz für nachgeschaltete Verfahren verwendet werden, was zu einer Energieeinsparung bei der Destillation führt.

Um den apparativen Aufwand und die Investitionskosten gering zu halten, werden erfindungsgemäß mehrere Reaktionsstufen bzw. Reaktionszellen und auch mehrere Zwischenkondensationen sowie Kühl- und Aufheizstufen in einem Reaktor realisiert. Verbindende Rohrleitungen werden möglichst vermieden, so dass sich Investitionskosten für Rohrleitungen und der Druckverlust reduzieren und die Beanspruchung der Rohrleitungen durch thermisch-mechanische Spannungen abnimmt. Die Prozessmedien werden möglichst innerhalb des Apparates von Prozessstufe zu Prozessstufe geführt.

### Bevorzugte Ausgestaltungen der Erfindung

Eine besondere Ausgestaltung des erfindungsgemäßen Verfahrens ist dadurch gekennzeichnet, dass das Rückführverhältnis Null ist. Durch die auf null reduzierte Kreisgasmenge reduziert sich neben der Reaktorgröße auch die Maße der Rohrleitungen erheblich. Ferner entfällt der bei den aus dem Stand der Technik bekannten Verfahren zur Methanolsynthese übliche Kreislaufverdichter und die erforderliche Kompressionsenergie verringert sich.

Durch die erhebliche, idealerweise bis auf null zurückgehende Reduzierung der Rückführungsgasmenge ergibt sich auch, dass die Menge und die Konzentration an akkumulierten Inertgasen, wie beispielsweise nicht umgesetztes Methan aus der Synthesegas-Erzeugung, im Synthesegaskreislauf deutlich verringert werden und damit den gesamten Synthesegaskreislauf mit Reaktorstufen, Kreislaufverdichter und weiteren Ausrüstungen entlastet. Als Optimum kann man einen fast vollständigen Umsatz pro Reaktordurchgang ansehen, bei dem Gaskreislauf verzichtet werden könnte und keine Akkumulierung von Inertgasen mehr auftritt. Ein derartiger Ansatz wäre auch für Einsatzgasgemische mit hohen Inertgasanteilen (z. B. hoher Stickstoffanteil bei Synthesegaserzeugung unter Verwendung von Luft) von besonderem Interesse, da bei Vorhandensein eines Gaskreislaufs die im Kreis zu führende Inertgasmenge stark ansteigen würde, was die Wirtschaftlichkeit des Verfahrens belastet.

Eine weitere vorteilhafte Ausgestaltung des erfindungsgemäßen Verfahrens sieht vor, dass die Katalysatormenge in den einzelnen Reaktionszonen (b) der einzelnen Reaktionszellen in Strömungsrichtung des Synthesegases durch den Reaktor abnimmt. Vergleichsversuche und Vergleichsrechnungen zeigen, dass unter diesen Bedingungen der Umsatz an Kohlenoxiden und die Raum-Zeit-Ausbeute in kg Methanol pro Liter Katalysatorvolumen und pro Stunde kg_{MoOH}/(Liter_{Kat} h) höher sind als bei ansteigender bzw. gleichbleibender Katalysatormenge in den einzelnen Reaktionszonen der einzelnen Reaktionszellen in Strömungsrichtung des Synthesegases.

Weitere Aspekte des erfindungsgemäßen Verfahrens sind dadurch gekennzeichnet, dass die Temperatur des Kühlmediums in den Reaktionszonen (b) der einzelnen Reaktionszellen zwischen 180 und 300 °C, bevorzugt zwischen 190 und 270 °C, meist bevorzugt zwischen 200 und 260 °C liegt und in Strömungsrichtung des Synthesegases durch den Reaktor gleich bleibt oder abnimmt Vergleichsversuche und Vergleichsrechnungen zeigen, dass unter diesen Bedingungen der Umsatz an Kohlenoxiden und die Raum-Zeit-Ausbeute in kg Methanol pro Liter Katalysator und pro Stunde kg_{MeOH}/(Liter_{Kat} h) höher sind als bei ansteigender Temperatur des Kühlmediums in den einzelnen Reaktionszonen der einzelnen Reaktionszellen in Strömungsrichtung des Synthesegases. Unter einem Gleichbleiben der Temperatur des Kühlmediums wird dabei eine Temperaturänderung von höchstens 5 K verstanden.

Besonders bevorzugt ist es in weiteren Aspekten der Erfindung, wenn bei dem erfindungsgemäßen Verfahren die Kondensationstemperatur in den Kühlzonen der einzelnen Reaktionszellen zwischen 20 und 120°C, bevorzugt zwischen 40 und 100 °C liegt und in Strömungsrichtung des Synthesegases durch den Reaktor gleich bleibt oder abnimmt. Vergleichsversuche und Vergleichsrechnungen zeigen, dass unter diesen Bedingungen der Umsatz an Kohlenoxiden und die Raum-Zeit-Ausbeute in kg Methanol pro Liter Katalysator und pro Stunde kg_{MeOH}/(Uter_{Kat} h) höher sind als bei ansteigender Kondensationstemperatur in Strömungsrichtung des Synthesegases.

In einem weiteren Aspekt des erfindungsgemäßen Verfahrens wird in allen Reaktionszellen der gleiche Wärmeträger verwendet und bei unterschiedlichen Druckstufen und zugehörigen Dampftemperaturen am jeweiligen Siedepunkt gearbeitet. Hierdurch ergeben sich logistische und technische Vorteile, da nur ein einziger Wärmeträger vorgehalten werden muss und Kreisläufe für die Zu- und Abfuhr weiterer Wärmeträger entfallen.

In einem weiteren Aspekt des erfindungsgemäßen Verfahrens sind alle Reaktionszellen mit demselben Dampferzeuger verbunden und der Wärmeträger wird in flüssiger Form bereitgestellt wird und erfährt im Bereich der Reaktionszellen zumindest eine Teilverdampfung. Auch hier ergeben sich Vorteile, da nur ein einziger Dampferzeuger vorgesehen werden muss. Da mit der Verdampfung eine besonders große Enthalpieänderung verbunden ist, kann die in den Reaktionszellen freigesetzte Reaktionswärme auf diese Weise besonders effektiv abgeführt werden.

Bevorzugt wird als Wärmeträger Wärmeträgeröl oder Wasser verwendet. Beide Wärmeträger sind gut handhabbar und leicht bzw. kostengünstig verfügbar.

### Ausführungsbeispiele

Weiterbildungen, Vorteile und Anwendungsmöglichkeiten der Erfindung ergeben sich auch aus der nachfolgenden Beschreibung von Ausführungsbeispielen und den Zeichnungen.

### Es zeigen:

Fig. 1 eine Reaktionszelle in einem Reaktor gemäß einer ersten Ausführungsform der Erfindung,
Fig. 2 eine Reaktionszelle in einem Reaktor gemäß einer zweiten Ausführungsform der Erfindung,
Fig. 3 eine Reaktionszelle in einem Reaktor gemäß einer dritten Ausführungsform der Erfindung,
Fig. 4 eine Reaktionszelle in einem Reaktor gemäß einer vierten Ausführungsform der Erfindung,
Fig. 5 ein erstes Beispiel für die Verschaltung zweier aufeinanderfolgender Reaktionszellen in einem erfindungsgemäßen Reaktor,
Fig. 6 ein zweites Beispiel für die Verschaltung zweier aufeinanderfolgender Reaktionszellen in einem erfindungsgemäßen Reaktor,
Fig. 7 ein Ausführungsbeispiel für die Verschaltung eine Reaktionszelle in einem erfindungsgemäßen Reaktor mit einem Dampferzeuger.

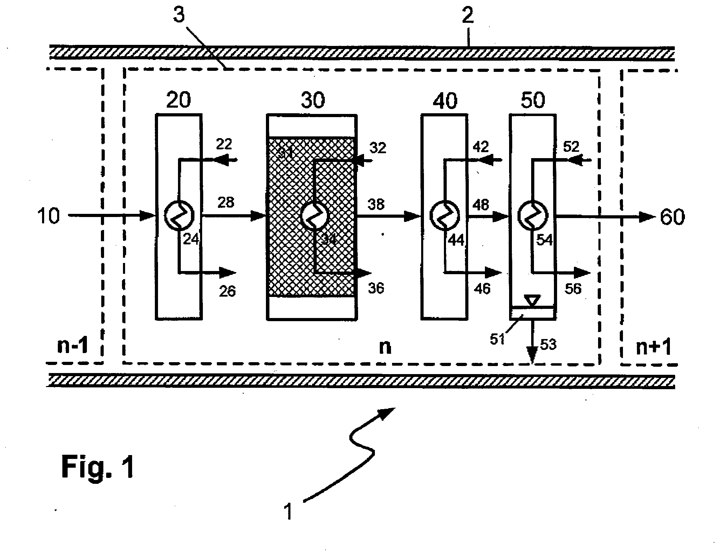

In Fig. 1 ist eine Reaktionszelle 3 in einem Reaktor 1 gemäß einer ersten Ausführungsform der Erfindung schematisch dargestellt. Die Reaktionszelle n befindet sich innerhalb des Reaktormantels 2, dessen Innenwand die physikalische Begrenzung des Reaktors nach außen bildet und den für die Durchführung der exothermen Gleichgewichtsreaktion gewählten Druck trägt.

Über Leitung 10 wird der Vorwärmzone 20 in der Reaktionszelle n der gasförmige, vorreagierte Produktstrom aus der vorgelagerten, stromaufwärts angeordneten Reaktionszelle n-1 zugeführt. Falls es sich bei der Reaktionszelle n um die erste Reaktionszelle in Strömungsrichtung handelt, wird über Leitung 10 das Einsatzgemisch zugeführt.

In der Vorwärmzone 20 wird der gasförmige Produktstrom oder das Einsatzgemisch auf die Reaktionstemperatur aufgeheizt. Dies erfolgt im indirekten Wärmetausch gegen ein Heizfluid, das über Leitung 22 dem Wärmetauscher 24 zugeführt wird und seinen Wärmeinhalt dort an den gasförmigen Produktstrom oder das Einsatzgemisch überträgt. Das abgekühlte Heizfluid wird über Leitung 26 aus dem Wärmetauscher abgeführt und in einer bildlich nicht dargestellten Heizvorrichtung aufgeheizt, um es erneut dem Wärmetauscher 24 zuzuführen.

Das aufgeheizte Einsatzgemisch bzw. der aufgeheizte, gasförmige Produktstrom wird über Leitung 28 der Reaktionszone 30 zugeführt, die eine Schüttung eines für die durchzuführende exotherme Gleichgewichtsreaktion aktiven Katalysators 31 und eine mit dem Katalysator in einer Wärmeaustauschbeziehung stehende Kühlvorrichtung 34 enthält. Die Abfuhr der durch die exotherme Reaktion freigesetzten Reaktionswärme erfolgt im indirekten Wärmetausch gegen ein Kühlfluid, welches optional teilweise verdampft, das über Leitung 32 dem Wärmetauscher 34 zugeführt und nach Aufnahme der in der Katalysatorschüttung freigesetzte Reaktionswärme über Leitung 36 abgeführt wird. Das aufgeheizte Kühlfluid wird in einer bildlich nicht dargestellten Kühlvorrichtung erneut abgekühlt, um es erneut dem Wärmetauscher 34 zuzuführen.

In der Reaktionszone erfolgt unter den gewählten Reaktionsbedingungen in der Katalysatorschüttung eine Teilumsetzung des Einsatzgemischs bzw. des gasförmigen Produktstroms aus der Reaktionszelle n-1 in einen mit kondensierbarem Reaktionsprodukt beladenen, gasförmigen Produktstrom, der über Leitung 38 aus der Reaktionszone abgeführt und einer ersten Kühlzone 40 zugeführt wird.

In der ersten Kühlzone 40 erfolgt eine Vorkühlung des mit kondensierbarem Reaktionsprodukt beladenen, gasförmigen Produktstroms, wobei bereits erste Anteile an Kondensat erhalten werden können, die über eine bildlich nicht dargestellte Abscheidevorrichtung und Leitungen aus dem Reaktor 1 ausgeleitet werden können. Alternativ kann die Vorkühlung in der ersten Kühlzone aber auch so durchgeführt werden, dass der Taupunkt des Gasstroms noch nicht unterschritten wird. Die Vorkühlung erfolgt im indirekten Wärmetausch gegen ein Kühlfluid, das über Leitung 42 dem Wärmetauscher 44 zugeführt und nach Wärmeaufnahme über Leitung 46 abgeführt wird. Das aufgeheizte Kühlfluid wird in einer bildlich nicht dargestellten Kühlvorrichtung erneut abgekühlt, um es erneut dem Wärmetauscher 44 zuzuführen.

Der vorgekühlte, aber immer noch mindestens mit einem Teil des kondensierbaren Reaktionsprodukts beladene, gasförmigen Produktstrom wird über Leitung 48 aus der ersten Kühlzone ausgeleitet und der zweiten Kühlzone 50 zugeführt. In der zweiten Kühlzone 50 erfolgt die weitere Abkühlung des mit kondensierbarem Reaktionsprodukt beladenen, gasförmigen Produktstroms, wobei dessen Taupunkt unterschritten wird. Hierbei wird ein flüssiges Kondensat erhalten, das mittels einer in die zweite Kühlzone integrierten Abscheidevorrichtung 51 vom Gasstrom abgetrennt und mittels Leitung 53 aus dem Reaktor ausgeleitet und der bildlich nicht dargestellten Produktaufarbeitung zugeführt wird. Die Abkühlung erfolgt im indirekten Wärmetausch gegen ein Kühlfluid, das über Leitung 52 dem Wärmetauscher 54 zugeführt und nach Wärmeaufnahme über Leitung 56 abgeführt wird. Das aufgeheizte Kühlfluid wird in einer bildlich nicht dargestellten Kühlvorrichtung erneut abgekühlt, um es erneut dem Wärmetauscher 54 zuzuführen.

Der abgekühlte und von Kondensat befreite, gasförmige Produktstrom wird über Leitung 60 aus der zweiten Kühlzone 50 und somit auch aus der Reaktionszelle n ausgeleitet. Er wird dann der stromabwärts angeordneten Reaktionszelle n+1 zugeführt, um eine weitere Umsetzung der gasförmigen Reaktanten zu Zielprodukten zu ermöglichen. Wenn keine weitere Umsetzung der gasförmigen Reaktanten erwünscht oder möglich ist, wird das verbliebene Restgas über Leitung 60 aus dem Reaktor ausgeleitet und der weiteren Aufarbeitung bzw. Entsorgung zugeführt. Alternativ kann der Restgasstrom nach Rückführen und Vermischen mit frischem Einsatzgemisch erneut dem Reaktor aufgegeben werden.

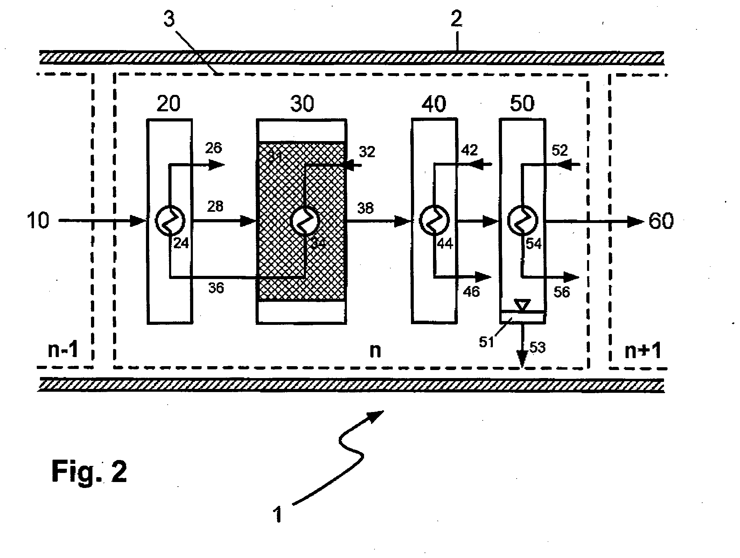

In den in Fig. 2 bis Fig. 7 schematisch dargestellten Ausgestaltungen entsprechen gleiche Bezugszeichen grundsätzlich den Vorrichtungsbestandteilen, wie sie bereits bei der Erläuterung der ersten Ausgestaltung der Erfindung, Fig. 1, beschrieben wurden. Auch die jeweiligen Arbeitsschritte und Verfahrensbedingungen sind gleich, sofern nicht nachfolgend anders beschrieben.

Im Unterschied zu der ersten Ausgestaltung wird in Fig. 2 das durch Aufnahme der Reaktionswärme in der Reaktionszone 30 aufgeheizte Kühlfluid über Leitung 36 zum Wärmetauscher 24 der Vorwärmzone 20 geführt und dort als Heizfluid für das Aufheizen des Einsatzgemischs oder des gasförmigen Produktstroms aus der vorgelagerten Reaktionszelle genutzt. Auf diese Weise wird die Wärmeintegration innerhalb des Reaktors verbessert. Diese Option ist besonders interessant, wenn man ein (teil-)verdampfendes Kühlmedium in der Reaktionszone 30 einsetzt und in der Vorwärmzone 20 zumindest teilweise wieder kondensiert und dort als Heizmedium nutzt. Bei einer vertikalen Anordnung von Vorwärmzone (oben) und darunter liegender Reaktionszone lässt sich dies einfach mit einer einzigen Anordnung, enthaltend eine obere Vorwärmzone ohne Katalysator und eine untere Reaktionszone mit Katalysator realisieren, die auf der Wärmeträgerseite direkt verbunden sind. Entstehender Dampf aus der Reaktionszone steigt auf und wird zumindest teilweise als Heizmedium in der Vorwärmzone genutzt, kondensierter Dampf fließt als Flüssigkeit zurück zur Reaktionszone. Das durch Wärmetausch mit dem in Leitung 10 zugeführten Gasstrom abgekühlte Heizfluid kann anschließend, optional nach weiterer Abkühlung in einer bildlich nicht dargestellten Kühlvorrichtung, als Kühlfluid über Leitung 32 zu dem Wärmetauscher 34 der Reaktionszone 30 zurückgeführt werden.

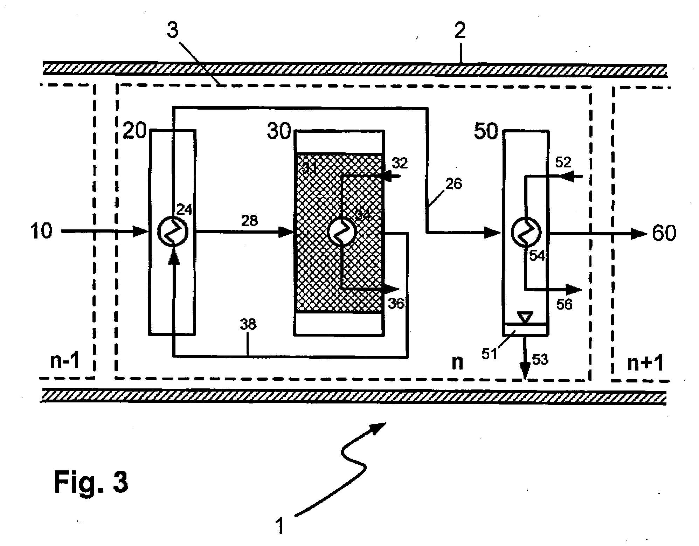

Im Unterschied zu der ersten Ausgestaltung wird in Fig. 3 der aus der Reaktionszone 30 über Leitung 38 ausgeleitete Produktstrom als Heizfluid zum Wärmetauscher 24 der Vorwärmzone 20 geführt und dient dort zum Aufheizen des über Leitung 10 herangeführten Einsatzgemischs bzw. gasförmigen Produktstroms aus der vorgelagerten Reaktionszelle. Die Vorwärmzone 20 und die erste Kühlzone 40 fallen somit zusammen. Auch auf diese Weise wird die Wärmeintegration innerhalb des Reaktors verbessert. Der durch Wärmetausch abgekühlte Produktstrom wird sodann über Leitung 26 zu der zweiten Kühlzone 50 geführt.

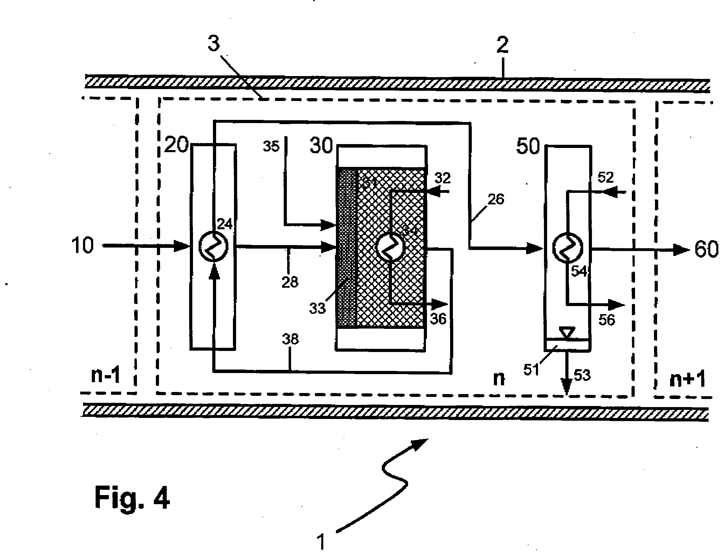

Im Unterschied zu der zuvor erörterten Ausgestaltung gemäß Fig. 3 enthält die Reaktionszone in Fig. 4 zwei Schüttungen von Katalysatoren 31, 33 mit unterschiedlicher Aktivität für die exotherme Gleichgewichtsreaktion, die nacheinander von dem Einsatzgemisch bzw. gasförmigen Produktstrom aus der vorgelagerten Reaktionszelle durchströmt werden. In der gezeigten Ausgestaltung wird nur die stromabwärts angeordnete Katalysatorschüttung 31 mittels der Kühlvorrichtung 34 gekühlt. Eine Ausgestaltungsmöglichkeit sieht vor, dass die Katalysatorschüttung 33 einen Katalysator enthält, der im Vergleich zu der Katalysatorschüttung 31 eine höhere Aktivität aufweist. Hierdurch kann die katalytische Umsetzung zunächst in Gang gesetzt werden und die dabei freigesetzte Wärmemenge trägt zu der Aufheizung des Reaktionsgemischs auf die gewählte Eintrittstemperatur in die Katalysatorschüttung 31 bei, wodurch der Wärmetauscher 24 in der Vorwärmzone 20 verkleinert werden kann. Für diese Funktion als Zündkatalysator reicht erfahrungsgemäß ein in Bezug auf die Haupt-Katalysatorschüttung 31 kleines bzw. kurzes Katalysatorbett aus. Die Reaktion in der Haupt-Katalysatorschüttung 31 verläuft dann gleichmäßiger, da Konzentrationsspitzen der Reaktanten bereits in der Katalysatorschüttung 33 reduziert werden und zudem die Katalysatorschüttung 31 gekühlt wird. Hierdurch wird die Bildung von Hot-Spots vermieden.

Alternativ kann in der Katalysatorschüttung 33 ein Katalysator eingesetzt werden, der im Vergleich zu der Katalysatorschüttung 31 eine geringere Aktivität aufweist. Dies empfiehlt sich besonders dann, wenn das Reaktionspotential des in die Reaktionszone eintretenden Gasgemischs hoch ist. Dies ist in der in Fig. 4 gezeigten Ausgestaltung der Fall, da über Leitung 35 der Reaktionszone 30 in der Reaktionszelle n frisches, also noch nicht vorreagiertes Einsatzgemisch zugeführt wird. Hierdurch wird die Reaktion langsamer und kontrollierter in Gang gesetzt und der Hauptteil der Reaktionswärme wird in der gekühlten Katalysatorschüttung 31 freigesetzt.

Das Zuführen frischen, noch nicht vorreagierten Einsatzgemischs zu Reaktionszellen mit n > 1 kann auch in Verbindung mit den anderen hier erörterten Ausgestaltungen des erfindungsgemäßen Reaktors sinnvoll sein. Ferner kann es vorteilhaft sein, frisches, noch nicht vorreagiertes Einsatzgemisch zu mehr als einer Reaktionszelle mit n > 1 zuzuführen.

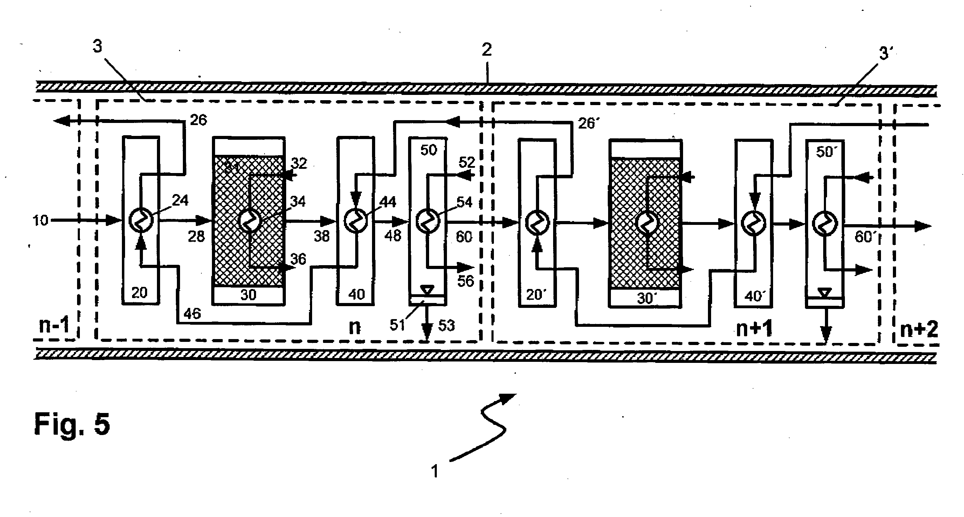

In der in Fig. 5 schematisch dargestellten Ausgestaltung ist eine Verschaltungsmöglichkeit zweier aufeinanderfolgender Reaktionszellen n und n+1 gezeigt. Entsprechende Vorrichtungsbestandteile der Reaktionszelle n+1 sind mit einem Apostroph an dem jeweiligen Bezugszeichen gekennzeichnet. Hierbei wird abgekühltes Heizfluid aus der Vorwärmzone 20' der Reaktionszelle n+1 über Leitung 26' dem Wärmetauscher 44 in der ersten Kühlzone 40 der Reaktionszelle n zugeführt und dient dort zur Vorkühlung des aus der Reaktionszone 30 über Leitung 38 abgeführten Gasstroms. Entsprechend wird abgekühltes Heizfluid aus der Vorwärmzone 20 der Reaktionszelle n über Leitung 26 dem entsprechenden Wärmetauscher in der ersten Kühlzone der Reaktionszelle n-1 zugeführt. Auf diese Weise wird eine noch weitergehende Wärmeintegration innerhalb des Reaktors erreicht, die sich nunmehr über mehrere Reaktionszellen erstreckt. Das im Wärmetauscher 44 aufgeheizte Heizfluid wird über Leitung 46 dem Wärmetauscher 24 zugeführt und dient dort zur Vorheizung des in die Reaktionszelle n über Leitung 10 eintretenden Gemischs.

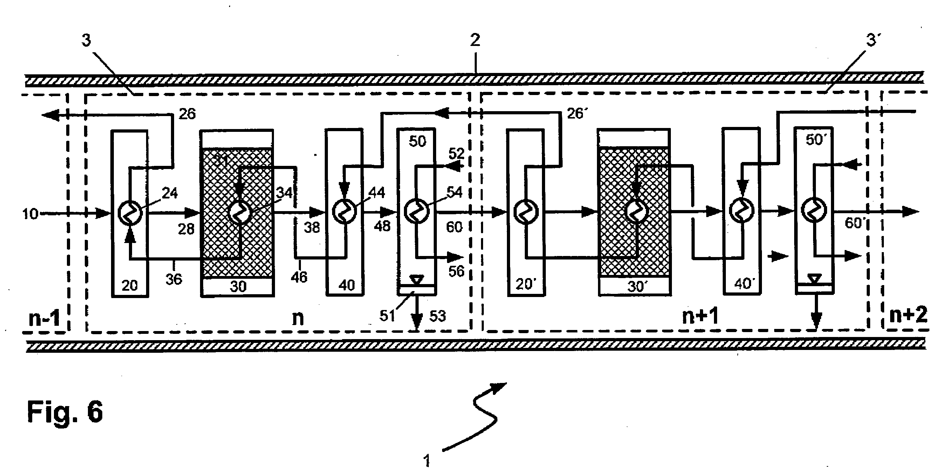

Im Unterschied zu der zuvor erörterten Ausgestaltung gemäß Fig. 5 wird in dem Ausführungsbeispiel der Fig. 6 zusätzlich das aus der jeweiligen ersten Kühlzone 40, 40' usw. abgeführte, aufgeheizte Kühlmittel dem Wärmetauscher der jeweils vorgeschalteten Reaktionszone 30, 30' usw. als Kühlmittel zugeführt. Das in der Reaktionszone weiter aufgeheizte Kühlmittel wird nachfolgend dem Wärmetauscher der jeweils vorgeschalteten Vorwärmzone als Heizfluid zugeführt. Diese Ausgestaltung kann sich insbesondere zur Durchführung exothermer Reaktionen mit mäßiger Wärmetönung eignen. Günstig ist im Rahmen dieser Ausgestaltung weiterhin die Verwendung eines Kühlfluids bzw. Heiz-fluids mit hoher Wärmeaufnahme- bzw. Wärmeabgabekapazität; hierfür eignen sich insbesondere Fluide, die bei ihrer Verwendung als Kühlfluid bzw. Heizfluid einen Phasenübergang flüssig - dampfförmig bzw. umgekehrt aufweisen. Schließlich kann es sinnvoll sein, die Reaktionszonen durch weitere, bildlich nicht dargestellte Kühlvorrichtungen zu kühlen, um eine intensivere Kühlwirkung und mehr Freiheitsgrade hinsichtlich der Temperaturführung in der Reaktionszone zu haben.

Bei den beiden zuletzt erörterten Ausgestaltungen kann es zudem sinnvoll sein, die aufgeheizten Kühlfluide bzw. abgekühlten Heizfluide zunächst zu einer oder mehrerer außerhalb des Reaktors angeordneten Kühl. bzw. Heizvorrichtungen zuzuführen, um die volle Wärmeaufnahme- bzw. Wärmeabgabekapazität des jeweiligen Fluids wiederherzustellen. Die Anordnung dieser externen Kühl- bzw. Heizvorrichtungen könnte beispielsweise im Leitungsweg der Leitungen 26,26' usw. (Aufheizen), 46, 46' usw. (Kühlen) bzw. 36, 36' usw. (Kühlen) erfolgen.

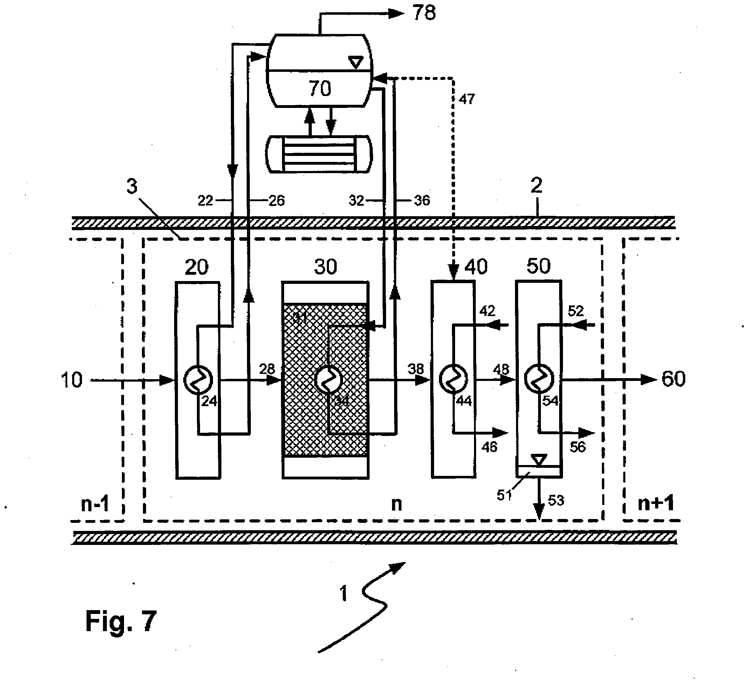

Die Verschaltung mit einer externen Kühl- bzw. Heizvorrichtung ist schematisch in der in Fig. 7 dargestellten Ausgestaltung gezeigt, bei der ein Dampferzeuger 70 außerhalb des Reaktors angeordnet ist. Diesem wird Heißkondensat entnommen und über Leitung 32 dem Wärmetauscher 34 der Reaktionszone 30 als Kühlmittel zugeführt, wobei es teilverdampft wird. Das resultierende Zweiphasengemisch flüssig - dampfförmig wird über Leitung 36 zum Dampferzeuger zurückgeführt.

Das Heißkondensat aus dem Dampferzeuger 70 kann ferner als Kühlmittel in der ersten Kühlzone 40 verwendet werden; dies ist schematisch durch die gestrichelte Leitung 47 dargestellt.

Dem Dampferzeuger 70 wird ferner Sattdampf entnommen, welcher über Leitung 22 dem Wärmetauscher 24 der Vorwärmzone 20 zugeführt wird. Durch die Wärmeabgabe an den über Leitung 10 herangeführten Strom erfolgt zumindest eine Teilkondensation. Der resultierende Strom kann entweder direkt über Leitung 26 zum Dampferzeuger zurückgeführt oder mittels anderer Vorrichtungen (bildlich nicht dargestellt) gesammelt werden und dann wieder zumindest teilweise zum Dampferzeuger zurückgeführt werden, um dort erneut verdampft zu werden.

In dem Ausführungsbeispiel der Fig. 7 kann ferner über eine Leitung 78 Sattdampf aus dem Dampferzeuger 70 abgeführt und als Exportdampf an externe Verbraucher abgegeben werden.

Als Wärmeträger bzw. Kühlmedium werden bevorzugt Medien eingesetzt, die sich in der Nähe ihres Siedepunktes befinden und daher leicht verdampfen (Kühlmedium) oder kondensieren (Wärmeträger, Heizmedium). Dies sichert eine gute Wärmeabfuhr durch den guten Wärmeübergang auf der Seite des verdampfenden bzw. kondensierenden Mediums und erlaubt eine präzise Temperaturregelung über den Druck. Um unterschiedliche Temperaturbedingungen in den verschiedenen Stufen einzustellen, wird der Druck auf der Seite des Wärmeträgers bzw. Kühlmediums individuell für jede Stufe geregelt. Mit zunehmender Katalysatorlaufzeit werden die Bedingungen mittels entsprechender Einstellung des Druckes auf der Kühlmediumseite angepasst und somit die Reaktionstemperatur nachgeführt, um den Umsatz entsprechend hoch zu halten.

Bezüglich der gewünschten Reaktionsbedingungen kann beispielsweise bei der Methanolsynthese Wasserdampf als Wärmeträger eingesetzt werden. Jedoch zeigt sich, dass bei Verwendung von Wasser für den gewünschten Temperaturbereich relativ große Druckunterschiede eingestellt werden müssen, um einen breiten Temperaturbereich abzudecken (z. B. 250°C: ca. 40 bar, 264 °C: ca. 50 bar). Benutzt man dagegen ein verdampfendes Wärmeträgeröl (z. B. Dowtherm A) in einem Kreislauf zur Wasserdampferzeugung, so kann man in einem sehr engen Druckbereich arbeiten und dennoch einen großen Temperaturbereich abdecken (Bsp.: 255 °C: 0,97 bar, 305 °C: 2,60 bar, entsprechend einem Temperaturbereich von 50 °C bei einem Druckunterschied von nur 1,6 bar. Dadurch kann man mit einer einfachen Wärmeträgeröl-Dampftrommel auf dem entsprechendem Anlagenniveau (ca. 20 bis 25 m) arbeiten und allein die Höhendifferenz nutzen, um die individuellen Druck- bzw. Temperaturbereiche einzustellen.

In den Abkühlzonen und/oder Kondensationszonen kann Kühlwasser oder auch ein verdampfender Wärmeträger eingesetzt werden, während in den Heizzonen ein kondensierender oder auch flüssiger Wärmeträger zum Einsatz kommen kann.

Bei vielen Ausgestaltungen, beispielsweise bei allen zuvor erörterten Ausgestaltungen, kann es vorteilhaft sein, die Wärmetransporträume durch jeweils wenigstens ein Thermoblech auszubilden. Unter den Wärmetransporträumen werden dabei die Bereiche des Reaktors verstanden, in denen es zum Wärmetausch zwischen der die Reaktanten bzw. Reaktionsprodukte enthaltenden Gasströmung und Heiz- bzw. Kühlfluiden kommt, also die Vorwärmzone, die Reaktionszone und die Kühlzonen.

Ein Thermoblech im Sinne der Erfindung besteht aus zwei Blechen, welche an den Rändern verbunden, vorzugsweise zusammengeschweißt sind und über deren Oberfläche eine Vielzahl von zusätzlichen Verbindungen, vorzugsweise Punktschweißungen, die die Platten ebenfalls miteinander verbinden, verteilt sind. Solche Platten können automatisiert von Robotern oder Maschinen und somit zu sehr günstigen Preisen hergestellt werden. Nach der Verschweißung werden die Bleche durch eine hydraulische Umformung, in der Regel das Einpressen einer Flüssigkeit unter hohem Druck, expandiert, wodurch kissenartige Kanäle zwischen den Blechen entstehen, durch die ein Heiz- oder Kühlfluid geleitet werden kann. Über die Wärmetransporträume kann daher bestimmten Bereichen des Reaktor durch das Durchleiten von Heiz- bzw. Kühlfluiden sowohl Wärmeenergie zu- als auch abgeführt werden.

Die Reaktionszonen können bei Verwendung von Thermoblechen so ausgestaltet werden, dass in dem Reaktor zunächst zwei Thermobleche im Wesentlichen parallel angeordnet werden. Im Wesentlichen parallel im Sinne der Erfindung bedeutet, dass die Ausrichtung der Thermobleche zueinander von der Parallelen maximal um +/- 20°, bevorzugt maximal um +/- 10°, besonders bevorzugt maximal um +/- 5°, ganz besonders bevorzugt maximal um +/- 2" voneinander abweicht. Danach kann der Freiraum zwischen den Thermoblechen mit einer Schüttung eines festen, körnigen, partikelförmigen oder pelletförmigen Katalysators aufgefüllt werden, wobei der seitliche Abschluss des sich ergebenden Katalysatorbettes durch Netze, Gitter, Lochbleche, Roste, Schüttungen von Inertmaterial und/oder die Reaktorinnenwand gebildet wird.

Besonders bevorzugt schließen sich parallel zu dieser Anordnung mindestens ein, bevorzugt mehrere weitere, beabstandete Thermobleche an, wobei sich insgesamt ein Plattenpaket ergibt und die Freiräume zwischen den Thermoblechen mit Katalysatorschüttungen aufgefüllt werden. Auf diese Weise wird in der Reaktionszone eine kompakte, sandwichartige Struktur mit einer intensiv wirkendenden, sich über die Länge der Reaktionszone erstreckenden Kühlvorrichtung erhalten. Die Beaufschlagung der einzelnen Katalysatorschüttungen mit dem Reaktionsgasgemisch erfolgt dabei parallel. Die Plattenpakete können, bezogen auf die mit Katalysator gefüllten Freiräume, parallel oder senkrecht zur Längsachse des Reaktors ausgerichtet werden.

Die Abstände zwischen den Thermoplatten werden gemäß der Wärmetönung der durchzuführenden Reaktion ausgewählt: Für stark exotherme Reaktionen wird dieser Abstand kleiner gewählt als für schwächer exotherme Reaktionen, Dabei werden in der ersten Reaktionszone kleinere Plattenabstände bevorzugt, da hier der größte Umsatz erzielt wird und die größte Wärmeabfuhr realisiert werden muss. Die Thermoblechplattenabstände der ersten Reaktionszone betragen im Falle der Methanolsynthese bevorzugt 20 bis 45 mm. Der Abstand bezieht sich dabei auf den Abstand von Mittellinie zur Mittellinie, d. h. der lichte Abstand zwischen den Platten fällt je nach Thermoblechdicke und Expansion des Hohlraumes entsprechend kleiner aus. Ferner wird der Abstand an die Abmessungen der Katalysatorpartikel angepasst, um eine optimale Wärmeabfuhr sowie ein gutes Schüttgutverhalten beim Befüllen und Entleeren des Katalysators ohne Brückenbildung sicherzustellen. In der zweiten und den nachfolgenden Reaktionszonen werden die Abstände üblicherweise größer gewählt.

Insbesondere bei horizontaler Anordnung des Reaktors bei gleichzeitig senkrechter Anordnung der Katalysatorschüttungen in den Reaktionszonen ergibt sich die Möglichkeit der einfachen Entfernung des Katalysators aus dem Reaktor zum Zwecke des Katalysatoraustauschs. Dabei sind zur Entleerung entsprechende Revisionsöffnungen im Reaktormantel vorzusehen, die beispielsweise durch einen Klapp- oder Schiebemechanismus betätigt werden. Der Schiebemechanismus kann sehr platzsparend ausgeführt werden, dabei ist es vorteilhaft, wenn sich die Tragroste der benachbarten Reaktionszonen mittels entsprechender Führungsschienen übereinander schieben lassen, so dass benachbarte Bereiche nacheinander entleert werden können.

In besonderer Ausgestaltung lassen sich sowohl stromabwärts als auch stromaufwärts der gekühlten Plattenpakete adiabate, also ungekühlte Reaktorbetten vorsehen. Das kann vor allem interessant werden, wenn nur noch ein Restumsatz erzielt werden soll und eine Kühlung der Reaktion aufgrund der geringen Wärmeentwicklung nicht mehr notwendig ist oder am Eintritt in eine Reaktionsstufe, wenn es vorteilhaft ist, eine schnelle Temperaturerhöhung zu erzielen, bevor die Reaktanten den gekühlten Bereich der Reaktionszone eintreten.

Auch bei der Ausgestaltung der Vorwärmzone und der Kühlzonen können Thermobleche vorteilhaft im Sinne eines Plattenwärmetauschers eingesetzt werden. Auf die Verwendung von Rohrendplatten, wie sie bei Rohrbündelwärmetauschem erforderlich sind, kann dabei verzichtet werden. Zudem werden logistische und fertigungstechnische Vorteile erhalten, da sich die Zahl unterschiedlicher Bauteile des Reaktors und damit die Komplexität des Apparates reduzieren.

Eine weitere Ausgestaltungsmöglichkeit wird durch die Ausgestaltung der Wärmetransporträume mittels Lamellenwärmetauschern (plate fin heat exchanger) alternativ oder zusätzlich zu der Verwendung von Thermoblechen ermöglicht.

### Zahlenbeispiele

### Vergleich mit aus dem Stand der Technik bekannten Reaktoren

In den nachfolgenden Tabellen werden Kenndaten zum Betrieb des Reaktors in dem erfindungsgemäßen Verfahren mit aus dem Stand der Technik bekannten Reaktoren für die heterogen-katalytische Synthese von Methanol aus Synthesegas verglichen.

Im ersten Vergleichsfall wird ein erfindungsgemäßes Verfahren mit einem Reaktor mit drei Reaktionszellen einem solchen mit dreistufigem technischen Reaktor gegenübergestellt, der zwei parallel geschaltete wassergekühlte Reaktoren WCR, stromabwärts gefolgt von einem gasgekühlten Reaktor GCR, umfasst. Die technische Anlage verfügt über keine Zwischenkondensation zwischen WCR und GCR. Das Einsatzgas ist in beiden Fällen hinsichtlich Zusammensetzung und Stoffmengenstrom gleich, es handelt sich um ein Synthesegas mit folgender Zusammensetzung: 8,4 Vol.-% CO₂, 20,1 Vol.-% CO, 68 Vol.-% H₂, Rest Inertkomponenten. Der Eintrittsdruck in den Reaktor beträgt jeweils 75 bar, Überdruck. In Tabelle 1 sind die wesentlichen Vergleichsdaten für beide Reaktoren zusammengestellt. Dabei bedeutet Xₚₚ(k) den Umsatz der Komponente k pro Durchgang durch den Reaktor (per pass) und Xₜₒₜ(k) ihren Gesamtumsatz über den Reaktor inklusive Gaskreislauf. STY ist die Raum-Zeit-Ausbeute an Methanol in kg/h, bezogen auf einen Liter Katalysatorvolumen.

Wie anhand der in Tabelle 1 zusammengestellten Daten zu erkennen ist, ist der Umsatz an Kohlenoxiden für den Gesamtreaktor in beiden Fällen vergleichbar; jedoch sind die Umsätze pro Reaktordurchgang für den erfindungsgemäßen Reaktor deutlich höher. Für letzteren sind zudem die Maximaltemperatur im Katalysatorbett, die Konzentration an Nebenprodukten und das benötigte Rückführverhältnis geringer.

**Tabelle 1: Vergleich der Kenndaten des erfindungsgemäßen Verfahrens mit Reaktor mit drei Reaktionszellen mit einem Verfahren mit dreistufigem Methanolsynthesereaktor (2 WCR parallel + GCR) gemäß Stand der Technik**

| | **Methanolsynthesereaktor mit 2 WCR parallel + GCR Vergleichsbeispiel** | **Reaktor mit drei Reaktionszellen Erfindung** |
|---|---|---|
| Xₚₚ(CO) / % | 81,9 | 95,5 |
| Xₚₚ(CO₂) / % | 28.0 | 60,7 |
| Xpp(COx) / % | 54,6 | 82,7 |
| Xₜₒₜ(CO) / % | 99.2 | 99,1 |
| Xₜₒₜ(CO₂) / % | 85,4 | 84,4 |
| Xₜₒₜ(COₓ) / % | 95,2 | 94,4 |
| STY(MeOH) / kg/(h Liter_{Kat}) | 0,86 | 1,26 |
| V_{Kat,tot}/ m³ | 315 | 180 |
| Tᵢₙ/°C | 230 | 215 |
| Tₘₐₓ / °C | 270 | 230 |
| Nebenprodukte / ppm | 6200 | 3250 |
| Rückführverhältnis | 2,2 | 0,5 |

In Tabelle 2 wird nachfolgend ein Verfahren mit einstufigem, wassergekühlten Reaktor zur Methanolsynthese mit einem erfindungsgemäßen Verfahren mit einem Reaktor verglichen, der vier Reaktionszellen umfasst, wobei das erfindungsgemäße Verfahren ohne Rückführung betrieben wird. Das Einsatzgas ist in beiden Fällen hinsichtlich Zusammensetzung und Stoffmengenstrom gleich, es handelt sich um ein Synthesegas mit folgender Zusammensetzung: 7 Vol.-% CO₂, 16 Vol.-% CO, 73 Vol.-% H₂, Rest Inertkomponenten. Der Eintrittsdruck in den Reaktor beträgt jeweils 75 bar, Überdruck. **Tabelle 2:** Vergleich der Kenndaten des erfindungsgemäßen Verfahrens mit Reaktor mit vier Reaktionszellen ohne Rückführung mit einem Verfahren mit einstufigem wassergekühlten Methanolsynthesereaktor

| | **Methanolsynthesereaktor (einstufiger gekühlter Reaktor mit hoher Gasrückführungsrate)** | **Reaktor mit vier Reaktionszellen ohne Gasrückführung** |
|---|---|---|
| | **Vergleichsbeispiel** | **Erfindung** |
| Xₚₚ(CO) / % | 90,8 | 99,7 |
| Xpp(CO₂) / % | 62,8 | 93,9 |
| Xpp(COx) / % | 80,6 | 97,8 |
| Xₜₒₜ(CO) / % | 99.2 | 99,7 |
| Xₜₒₜ(CO₂) / % | 94.7 | 93,9 |
| Xₜₒₜ(COₓ) / % | 97,9 | 97,8 |
| STY(MeOH) / kg/(h Liter_{Kat}) | 0,98 | 1,15 |
| **Rückführverhältnis** | 3,5 | 0 |

Mit dem erfindungsgemäßen Verfahren mit Reaktor mit vier Reaktionszellen wird ohne Rückführung eine um rund 15 % höhere Raum-Zeit-Ausbeute an Methanol erhalten. Insbesondere der CO₂-Umsatz pro Reaktordurchgang ist deutlich höher als bei dem Vergleichsbeispiel.

### Optimierung der Verfahrensbedingungen

In den nachfolgenden Tabellen wird der Einfluss bestimmter Verfahrensparameter in den einzelnen Reaktionszellen bei der heterogen-katalytischen Synthese von Methanol aus Synthesegas aufgezeigt. Die sonstigen Verfahrensbedingungen entsprechen denen des in Tabelle 2 dargestellten Beispiels (sog. Referenz in Tabellen 3 bis 5).

**Tabelle 3: Variation der Verteilung des Katalysatorvolumens**

| Fall | V_{Kat} / m³ | | | | | Xₜₒₜ(COₓ) / % | STY(MeOH) / kg/(h l_{Kat}) |
|---|---|---|---|---|---|---|---|
| | 1.Stufe | 2.Stufe | 3.Stufe | 4.Stufe | gesamt | gesamt | gesamt |
| Referenz | 8 | 8 | 8 | 8 | 32 | 95,1 | 1,53 |
| 1 | 4 | 6 | 10 | 12 | 32 | 92,1 | 1,49 |
| 2 | 12 | 10 | 6 | 4 | 32 | 96 | 1,56 |

**Tabelle 4: Variation der Kühltemperatur T_{cool} im Katalysatorbett**

| **Fall** | **T_{cool} / °C** | | | | **Xₜₒₜ(COₓ) / %** | **STY(MeOH) / kg/(h l_{Kat})** |
|---|---|---|---|---|---|---|
| | 1. Stufe | 2. Stufe | 3. Stufe | 4. Stufe | gesamt | gesamt |
| Referenz | 220 | 220 | 220 | 220 | 95,1 | 1,53 |
| 3 | 200 | 220 | 240 | 260 | 89,4 | 1,44 |
| 4 | 260 | 240 | 220 | 200 | 96 | 1,55 |

**Tabelle 5: Variation der Kondensationstemperatur T_{cond}**

| **Fall** | **T_{cond} / °C** | | | | **Kühlleistung / MW** | **CO₂-Verluste / %** |
|---|---|---|---|---|---|---|
| | 1. Stufe | 2. Stufe | 3. Stufe | 4. Stufe | gesamt | gesamt |
| Referenz | 40 | 40 | 40 | 40 | 39,9 | 7,4 |
| 5 | 100 | 80 | 60 | 40 | 33,7 | 3,7 |
| 6 | 40 | 60 | 80 | 100 | 34,9 | 6.0 |

### Gewerbliche Anwendbarkeit

Mit der Erfindung wird ein Reaktor zum Durchführen exothermer Gleichgewichtsreaktionen, insbesondere für die Durchführung der Methanolsynthese durch heterogen-katalysierte Umsetzung von Synthesegas vorgeschlagen, der es ermöglicht, die Reaktionsbedingungen entlang der Längskoordinate des Reaktors nachzuführen und somit zu optimieren, was beispielsweise im Falle der Methanolsynthese zu einer Reduzierung des Rückführverhältnis auf kleinere Werte führt, wie sie bei Verwendung der aus dem Stand der Technik bekannten Reaktoren bekannt sind. Entsprechende Rückführleitungen, Kreislaufverdichter usw. können daher kleiner ausgestaltet werden oder es kann gegebenenfalls ganz auf sie verzichtet werden. Hierdurch reduzieren sich die entsprechenden Investitionskosten.

Durch die Optimierung der Reaktionsbedingungen entlang der Längskoordinate des Reaktors wird ferner die Bildung unerwünschter Nebenprodukte reduziert, wodurch ein reineres Zielprodukt erhalten wird und sich der Reinigungsaufwand verringert.

### Bezugszeichenliste

- [1]: Reaktor
- [2]: Reaktormantel
- [3]: Reaktionszelle
- [10]: Leitung
- [20]: Vorwärmzone
- [22]: Leitung
- [24]: Wärmetauscher
- [26]: Leitung
- [28]: Leitung
- [30]: Reaktionszone
- [31]: Katalysatorbett
- [32]: Leitung
- [33]: Katalysatorbett
- [34]: Wärmetauscher
- [35]: Leitung
- [36]: Leitung
- [38]: Leitung
- [40]: erste Kühlzone
- [42]: Leitung
- [44]: Wärmetauscher
- [46]: Leitung
- [47]: Leitung
- [48]: Leitung
- [50]: zweite Kühl- und Abscheidezone
- [51]: Abscheidevorrichtung
- [52]: Leitung
- [53]: Leitung
- [54]: Wärmetauscher
- [56]: Leitung
- [60]: Leitung
- [70]: Dampferzeuger
- [78]: Leitung

## Patentansprüche

1. Verfahren zum Herstellen von Methanol durch Umsetzen eines Wasserstoff und Kohlenoxide enthaltenden Synthesegaseinsatzes, umfassend folgende Verfahrensschritte:
(a) Bereitstellen eines Reaktors, umfassend mehrere Reaktionszellen in einem Reaktormantel, wobei jede Reaktionszelle die folgenden, hintereinander geschalteten und in Fluidverbindung miteinander stehenden Baugruppen umfasst:
(aa) eine Vorwärmzone, geeignet zum Aufheizen des Einsatzgemischs oder des gasförmigen Produktstroms aus der vorgelagerten Reaktionszelle, wobei die Vorwärmzone in der ersten Reaktionszelle in Strömungsrichtung des gasförmigen Einsatzgemischs optional entfallen kann,
(ab) mindestens eine Reaktionszone, enthaltend einen für die durchzuführende exotherme Gleichgewichtsreaktion aktiven Katalysator als Katalysatorschüttung in einem Katalysatorbett und eine mit dem Katalysatorbett in einer Wärmeaustauschbeziehung stehende Kühlvorrichtung,
(ac) mindestens eine Kühlzone, enthaltend eine Kühlvorrichtung, geeignet zur Abkühlung des aus der Reaktionszone austretenden, teil umgesetzten und mit kondensierbarem Reaktionsprodukt beladenen, gasförmigen Produktstroms auf eine Temperatur unterhalb des Taupunktes dieses Gases,
(ad) eine Abscheidezone, enthaltend eine Phasentrennvorrichtung zum Auftrennen des aus der Kühlzone austretenden Produktstroms in einen von Kondensat befreiten, gasförmigen Produktstrom und einen flüssiges Reaktionsprodukt umfassenden Kondensatstrom,
(ae) Mittel zum Ausleiten des flüssiges Reaktionsprodukt umfassenden Kondensatstroms und optional Mittel zum Zuführen des Kondensatstroms zu einer Aufarbeitungsvorrichtung für das Reaktionsprodukt,
(af) Mittel zum Ausleiten des von Kondensat befreiten, gasförmigen Produktstroms und Mittel zum Zuführen dieses gasförmigen Produktstroms zu einer nachgelagerten, stromabwärts angeordneten Reaktionszelle oder Mittel zum Ausleiten des gasförmigen Produktstroms aus dem Verfahren,
(b) Bereitstellen eines Wasserstoff und Kohlenoxide enthaltenden Synthesegaseinsatzes und Einleiten desselben in den Reaktor,
(c) Mindestens teilweises Umsetzen des Synthesegaseinsatzes in dem Reaktor unter Methanolumsetzungsbedingungen,
(d) Ausleiten eines Methanol und Wasser umfassenden, flüssigen Reaktorproduktstroms aus dem Reaktor und optional Zuführen des flüssigen Reaktorproduktstroms zu einer weiteren Abscheidevorrichtung und/oder mindestens einer weiteren Methanol-Aufarbeitungsvorrichtung,
(g) Ausleiten eines Synthesegas-Abstroms und Rückführen dieses Synthesegas-Abstroms zu dem Reaktor mit einem festgelegten Rückführverhältnis und/oder Ausleiten des Synthesegas-Abstroms aus dem Verfahren.

2. Verfahren nach Anspruch 1, **dadurch gekennzeichnet, dass** das Rückführverhältnis Null ist.

3. Verfahren nach Anspruch 1 oder 2, **dadurch gekennzeichnet, dass** die Katalysatormenge in den einzelnen Reaktionszonen (ab) der einzelnen Reaktionszellen in Strömungsrichtung des Synthesegases durch den Reaktor abnimmt.

4. Verfahren nach einem der vorigen Ansprüche, **dadurch gekennzeichnet, dass** die Temperatur des Kühlmediums in den Reaktionszonen (b) der einzelnen Reaktionszellen zwischen 180 und 300 °C liegt und in Strömungsrichtung des Synthesegases durch den Reaktor gleich bleibt oder abnimmt.

5. Verfahren nach einem der vorigen Ansprüche, **dadurch gekennzeichnet, dass** die Temperatur des Kühlmediums in den Reaktionszonen (b) der einzelnen Reaktionszellen zwischen 190 und 270 °C liegt und in Strömungsrichtung des Synthesegases durch den Reaktor gleich bleibt oder abnimmt.

6. Verfahren nach einem der vorigen Ansprüche, **dadurch gekennzeichnet, dass** die Temperatur des Kühlmediums in den Reaktionszonen (b) der einzelnen Reaktionszellen zwischen 200 und 260 °C liegt und in Strömungsrichtung des Synthesegases durch den Reaktor gleich bleibt oder abnimmt.

7. Verfahren nach einem der vorigen Ansprüche, **dadurch gekennzeichnet, dass** die Kondensationstemperatur in den Kühlzonen der einzelnen Reaktionszellen zwischen 20 und 120 °C liegt und in Strömungsrichtung des Synthesegases durch den Reaktor gleich bleibt oder abnimmt.

8. Verfahren nach einem der vorigen Ansprüche, **dadurch gekennzeichnet, dass** die Kondensationstemperatur in den Kühlzonen der einzelnen Reaktionszellen zwischen 40 und 100 °C liegt und in Strömungsrichtung des Synthesegases durch den Reaktor gleich bleibt oder abnimmt.

9. Verfahren nach einem der vorigen Ansprüche, **dadurch gekennzeichnet, dass** in allen Reaktionszellen der gleiche Wärmeträger verwendet wird und bei unterschiedlichen Druckstufen und zugehörigen Dampftemperaturen am jeweiligen Siedepunkt arbeitet.

10. Verfahren nach einem der vorigen Ansprüche, **dadurch gekennzeichnet, dass** alle Reaktionszellen mit demselben Dampferzeuger verbunden sind und der Wärmeträger in flüssiger Form bereitgestellt wird und im Bereich der Reaktionszellen zumindest eine Teilverdampfung erfährt.

11. Verfahren nach einem der vorigen Ansprüche, **dadurch gekennzeichnet, dass** der Wärmeträger Wärmeträgeröl oder Wasser ist.

## Claims

1. Process for preparing methanol by converting a synthesis gas feed comprising hydrogen and carbon oxides, comprising the following process steps:
(a) providing a reactor comprising multiple reaction cells in a reactor shell, wherein each reaction cell comprises the following series-connected assemblies that are in fluid connection with one another:
(aa) a preheating zone suitable for heating the feed mixture or the gaseous product stream from the upstream reaction cell, wherein the preheating zone can optionally be dispensed with in the first reaction cell in flow direction of the gaseous feed mixture,
(ab) at least one reaction zone comprising a catalyst active in respect of the exothermic equilibrium reaction to be conducted in the form of a catalyst packing in a catalyst bed, and a cooling apparatus in a heat-exchanging relationship with the catalyst bed,
(ac) at least one cooling zone comprising a cooling apparatus suitable for cooling the partly converted gaseous product stream that has been laden with condensable reaction product and exits from the reaction zone to a temperature below the dew point of this gas,
(ad) a deposition zone comprising a phase separation apparatus for separation of the product stream that exits from the cooling zone into a gaseous product stream that has been freed of condensate and a condensate stream comprising liquid reaction product,
(ae) means of discharging the condensate stream comprising liquid reaction product and optionally means of feeding the condensate stream to a workup apparatus for the reaction product,
(af) means of discharging the gaseous product stream that has been freed of condensate and means of feeding this gaseous product stream to a subsequent reaction cell arranged downstream or means of discharging the gaseous product stream from the process,
(b) providing a synthesis gas feed comprising hydrogen and carbon oxides and introducing it into the reactor,
(c) at least partly converting the synthesis gas feed in the reactor under methanol conversion conditions,
(d) discharging a liquid reactor product stream comprising methanol and water from the reactor and optionally feeding the liquid reactor product stream to a further deposition apparatus and/or at least one further methanol workup apparatus,
(g) discharging a synthesis gas output stream and recycling this synthesis gas output stream to the reactor with a fixed recycle ratio and/or discharging the synthesis gas output stream from the process.

2. Process according to Claim 1, **characterized in that** the recycle ratio is zero.

3. Process according to Claim 1 or 2, **characterized in that** the amount of catalyst in the individual reaction zones (ab) of the individual reaction cells decreases in flow direction of the synthesis gas through the reactor.

4. Process according to any of the preceding claims, **characterized in that** the temperature of the cooling medium in the reaction zones (b) of the individual reaction cells is between 180 and 300°C, and remains the same or decreases in flow direction of the synthesis gas through the reactor.

5. Process according to any of the preceding claims, **characterized in that** the temperature of the cooling medium in the reaction zones (b) of the individual reaction cells is between 190 and 270°C, and remains the same or decreases in flow direction of the synthesis gas through the reactor.

6. Process according to any of the preceding claims, **characterized in that** the temperature of the cooling medium in the reaction zones (b) of the individual reaction cells is between 200 and 260°C, and remains the same or decreases in flow direction of the synthesis gas through the reactor.

7. Process according to any of the preceding claims, **characterized in that** the condensation temperature in the cooling zones of the individual reaction cells is between 20 and 120°C, and remains the same or decreases in flow direction of the synthesis gas through the reactor.

8. Process according to any of the preceding claims, **characterized in that** the condensation temperature in the cooling zones of the individual reaction cells is between 40 and 100°C, and remains the same or decreases in flow direction of the synthesis gas through the reactor.

9. Process according to any of the preceding claims, **characterized in that** the same heat carrier is used in all reaction cells and the temperature employed is the respective boiling point at different pressure levels and corresponding vapour temperatures.

10. Process according to any of the preceding claims, **characterized in that** all reaction cells are connected to the same steam generator and the heat carrier is provided in liquid form and undergoes at least partial evaporation in the region of the reaction cells.

11. Process according to any of the preceding claims, **characterized in that** the heat carrier is heat carrier oil or water.

## Revendications

1. Procédé pour la production de méthanol par conversion d'une charge de gaz de synthèse contenant de l'hydrogène et des oxydes de carbone, comprenant les étapes de processus suivantes :
(a) fourniture d'un réacteur, comprenant plusieurs cellules de réaction dans une enveloppe de réacteur, chaque cellule de réaction comprenant les groupes de montage suivant, montés en série et se trouvant en communication fluidique entre eux :
(aa) une zone de préchauffage, appropriée pour le chauffage du mélange de charge ou du courant de produit gazeux provenant de la cellule de réaction précédente, la zone de préchauffage pouvant en option être absente dans la première cellule de réaction dans le sens de flux du mélange de charge gazeux,
(ab) au moins une zone de réaction, contenant un catalyseur actif pour la réaction équilibrée exothermique à effectuer, sous forme de garnissage de catalyseur dans un lit de catalyseur et un dispositif de refroidissement se trouvant en une relation d'échange thermique avec le lit de catalyseur,
(ac) au moins une zone de refroidissement, contenant un dispositif de refroidissement, appropriée pour le refroidissement du courant de produit gazeux sortant de la zone de réaction, partiellement converti et chargé de produit de réaction condensable, à une température inférieure au point de rosée de ce gaz,
(ad) une zone de séparation, contenant un dispositif de séparation de phases destiné à la séparation du courant de produit, sortant de la zone de refroidissement, en un courant de produit gazeux privé de condensat et un courant de condensat comprenant du produit de réaction liquide,
(ae) un moyen destiné à l'évacuation du courant de condensat comprenant du produit de réaction liquide et en option un moyen destiné à l'envoi du courant de condensat à un dispositif de traitement final pour le produit de réaction,
(af) un moyen destiné à l'évacuation du courant de produit gazeux, privé de condensat et un moyen destiné à l'envoi de ce courant de produit gazeux à une cellule de réaction suivante, disposée en aval, ou un moyen destiné à l'évacuation du courant de produit gazeux hors du processus,
(b) fourniture d'une charge de gaz de synthèse contenant de l'hydrogène et des oxydes de carbone et introduction de celle-ci dans le réacteur,
(c) au moins conversion partielle de la charge de gaz de synthèse dans le réacteur dans des conditions de conversion en méthanol,
(d) évacuation hors du réacteur d'un courant liquide de produit du réacteur, comprenant du méthanol et de l'eau et en option envoi du courant liquide de produit du réacteur à un autre dispositif de séparation et/ou à au moins un autre dispositif de traitement final du méthanol,
(g) évacuation d'un courant effluent de gaz de synthèse et renvoi de ce courant effluent de gaz de synthèse au réacteur à un rapport de recyclage fixé et/ou évacuation du courant effluent de gaz de synthèse hors du processus.

2. Procédé selon la revendication 1, **caractérisé en ce que** le rapport de recyclage est zéro.

3. Procédé selon la revendication 1 ou 2, **caractérisé en ce que** la quantité de catalyseur dans les zones de réaction individuelles (ab) des cellules de réaction individuelles dans le sens de flux du gaz de synthèse diminue le long du réacteur.

4. Procédé selon l'une quelconque des revendications précédentes, **caractérisé en ce que** la température du fluide de refroidissement dans les zones de réaction (b) des cellules de réaction individuelles se situe entre 180 et 300 °C et reste constante ou diminue dans le sens de flux du gaz de synthèse le long du réacteur.

5. Procédé selon l'une quelconque des revendications précédentes, **caractérisé en ce que** la température du fluide de refroidissement dans les zones de réaction (b) des cellules de réaction individuelles se situe entre 190 et 270 °C et reste constante ou diminue dans le sens de flux du gaz de synthèse le long du réacteur.

6. Procédé selon l'une quelconque des revendications précédentes, **caractérisé en ce que** la température du fluide de refroidissement dans les zones de réaction (b) des cellules de réaction individuelles se situe entre 200 et 260 °C et reste constante ou diminue dans le sens de flux du gaz de synthèse le long du réacteur.

7. Procédé selon l'une quelconque des revendications précédentes, **caractérisé en ce que** la température de condensation dans les zones de refroidissement des cellules de réaction individuelles se situe entre 20 et 120 °C et reste constante ou diminue dans le sens de flux du gaz de synthèse le long du réacteur.

8. Procédé selon l'une quelconque des revendications précédentes, **caractérisé en ce que** la température de condensation dans les zones de refroidissement des cellules de réaction individuelles se situe entre 40 et 100 °C et reste constante ou diminue dans le sens de flux du gaz de synthèse le long du réacteur.

9. Procédé selon l'une quelconque des revendications précédentes, **caractérisé en ce que** dans toutes les cellules de réaction le même fluide caloporteur est utilisé et à des niveaux de pression différents et aux températures de vapeur correspondantes fonctionne au point d'ébullition respectif.

10. Procédé selon l'une quelconque des revendications précédentes, **caractérisé en ce que** toutes les cellules de réaction sont connectées au même générateur de vapeur et le fluide caloporteur est fourni sous forme liquide et subit dans la zone des cellules de réaction au moins une vaporisation partielle.

11. Procédé selon l'une quelconque des revendications précédentes, **caractérisé en ce que** le fluide caloporteur est une huile caloporteuse ou l'eau.
